(19) Europäisches Patentamt
European Patent Office
Office européen des brevets

(11) Veröffentlichungsnummer: **0 053 819**
**B1**

# (12) EUROPÄISCHE PATENTSCHRIFT

(45) Veröffentlichungstag der Patentschrift: **28.05.86**

(51) Int. Cl.$^4$: **C 07 C 85/06,** C 07 C 85/24

(21) Anmeldenummer: **81110160.9**

(22) Anmeldetag: **04.12.81**

## (54) Verfahren zur Herstellung von cycloaliphatischen und/oder aromatischen Aminen.

(30) Priorität: **04.12.80 DE 3045719**

(43) Veröffentlichungstag der Anmeldung:
**16.06.82 Patentblatt 82/24**

(45) Bekanntmachung des Hinweises auf die Patenterteilung:
**28.05.86 Patentblatt 86/22**

(84) Benannte Vertragsstaaten:
**AT CH DE FR GB IT LI NL SE**

(56) Entgegenhaltungen:
**EP-A-0 022 751**
**EP-A-0 031 859**
**FR-A-1 427 543**
**FR-A-1 506 010**
**FR-A-1 530 477**
**US-A-3 347 921**
**US-A-3 361 818**
**US-A-3 931 298**

(73) Patentinhaber: **BASF Aktiengesellschaft**
**Carl-Bosch-Strasse 38**
**D-6700 Ludwigshafen (DE)**

(72) Erfinder: **Goetz, Norbert, Dr.**
**Schoefferstrasse 25**
**D-6520 Worms (DE)**
Erfinder: **Jacobs, Peter, Dr.**
**Kalmitstrasse 2**
**D-6718 Gruenstadt (DE)**
Erfinder: **Hupfer, Leopold, Dr.**
**Waltershoehe 3**
**D-6701 Friedelsheim (DE)**
Erfinder: **Toussaint, Herbert, Dr.**
**Knietschstrasse 11**
**D-6710 Frankenthal (DE)**
Erfinder: **Reiss, Wolfgang, Dr.**
**Bannwasserstrasse 70**
**D-6700 Ludwigshafen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 053 819 B1

**Beschreibung**

Die Erfindung betrifft ein Verfahren zur Herstellung von cycloaliphatischen und/oder aromatischen Aminen (Anilinen) aus entsprechenden aromatischen Hydroxyverbindungen (Phenolen).

Die älteste und zur Zeit immer noch wichtigste Methode zur Herstellung cycloaliphatischer Amine ist die Reduktion der entsprechenden Nitroverbindungen (Houben-Weyl "Methoden der Org. Chemie", Band 11/1, S. 360 ff) zu primären aromatischen Aminen und deren Hydrierung. Nachteilig ist dabei die Tatsache, daß die Nitrierung bei substituierten Aromaten oft nicht einheitlich verläuft und fast stets Isomerengemische anfallen.

Prinzipiell gibt es auch die Möglichkeit, Phenole durch Partialhydrierung in Cyclohexanone zu überführen (DE—AS 1 124 487, DE—AS 1 298 098, DE—AS 1 144 267, US—PS 3 124 614, CH—PS 463 493, DE—OS 2 045 882) und die Cyclohexanone mit Ammoniak und Wasserstoff zu Cyclohexylaminen umzusetzen (Houben-Weyle, loc.cit., S. 611—617).

Diese Wege sind aber, da sie mehrere unabhängige Verfahrensschritte erfordern, recht umständlich und nicht besonders wirtschaftlich.

Die einstufige (direkte) Überrführung von unsubstituiertem Phenol mit Ammoniak und Wasserstoff in Cyclohexylamin gelingt nach bisheriger Kenntnis in Gegenwart von Ruthenium- oder Rhodiumkatalysatoren (JP—OS 77/34 677, FR—PS 1 427 543, GB—PS 1 031 169, DE—PS 12 76 032).

In der US—PS 3 931 298 wird die Umsetzung von Phenolen zu Anilinen mittels eines Palladium- oder Platin-Tierkohle-Katalysators unter Cyclohexanon-Cokatalyse beschrieben. Das Verfahren, das in der Flüssigphase abläuft, benötigt aber lange Reaktionszeiten (bis zu 24 Std.).

Nach einer nicht vorveröffentlichten Beschreibung ist 2,6-Dialkylphenol schon mit Ammoniak und Wasserstoff im Bereich eines bestimmten geringen Druckes (7 bis 21 bar) in das entsprechende Cyclohexylamin überführt worden, wobei Platin- oder Palladiumkatalysatoren empfohlen wurden (EP—A 22 751).

Schwierigkeiten bieten bisher offenbar besonders Verfahren, um substituierte Phenole, z.B. das vorerwähnte 2,6-Dimethylphenol in entsprechende cycloaliphatische und insbesondere aromatische Amine zu überführen, die technisch wichtige Grundstoffe z.B. zur Herstellung von Pflanzenschutzmitteln darstellen.

Das europäische Patent Nr. 53 817 beschreibt ein Verfahren zur Herstellung von cycloaliphatischen Aminen durch Umsetzung von entsprechenden Phenolen mit Ammoniak und Wasserstoff bei erhöhter Temperatur, wobei man die Umsetzung in Gegenwart eines auf einem Träger aufgebrachten Ruthenium-, Rhodium-, Palladium- oder Platinkatalysators durchführt und einen Wasserstoffpartialdruck von wenigstens 20 bar bzw. einen Gesamtdruck von wenigstens 50 bar einhält.

Gegenstand des europäischen Patents Nr. 53 696 ist ein Verfahren zur Herstellung von cycloaliphatischen und/oder aromatischen Aminen durch Umsetzung von entsprechenden Phenolen mit Ammoniak und Wasserstoff, wobei man die Umsetzung in Gegenwart eines auf einem Träger aufgebrachten Palladiumkatalysators durchführt, der einen basischen Stoff und/oder ein Element aus der Gruppe 1 B, 2 B, 7 B der Periodensystems, Eisen, Kobalt oder Nickel enthält.

Die Aufgabe, cycloaliphatische und vor allem aromatische Amine aus entsprechenden Phenolen herzustellen, wird erfindungsgemäß im wesentlichen dadurch gelöst, daß man das Phenol mit Ammoniak und Wasserstoff an einem Edelmetall-(Platinmetall-)trägerkatalysator aus der Gruppe von Rhodium, Ruthenium und Platin, der einen basischen Stoff und/oder ein Element aus der Gruppe 1 B, 2 B, 7 B des Periodensystems, Eisen, Kobalt oder Nickel enthält unter im wesentlichen atmosphärischen Druck, z.b. bis zu 10 bar umsetzt, wobei die Umsetzung im wesentlichen in der Gasphase stattfindet.

Unter diesen Umständen ist die für die Benutzung der Erfindung erforderliche Temperatur bemerkenswert niedrig: In vielen Fällen genügt eine Temperatur von 100°C, um eine befriedigende Reaktionsgeschwindigkeit zu erreichen. Temperaturen über etwa 250°C sind nur selten erforderlich.

Durch Wahl der Bedingungen kann der Reaktionsablauf so gesteuert werden, daß ausschließlich gesättigte Cyclohexylamine oder überwiegend entsprechende aromatische Amine (Aniline) gebildet werden: Während bei atmosphärischem Druck und Verwendung von Palladium oder Platin die entsprechenden aromatischen Amine begünstigt sind, ist ein Betrieb unter mäßigem Druck zu bevorzugen, wenn die cycloaliphatischen Amine erhalten werden sollen. Temperatursteigerung begünstigt die Bildung der Aniline, Erhöhung des Wasserstoffdruckes wirkt im Sinne einer Bevorzugung der Cyclohexylamine, so daß im unteren Temperaturbereich (150 bis 230°C) in erster Linie die gesättigten Amine, im oberen Temperaturbereich (180 bis 300°C) hauptsächlich Aniline anfallen. Die erhaltenen Cyclohexylamine lassen sich bei Bedarf leicht in Aniline überführen.

Zur Gewinnung ein- oder mehrkerniger, cycloaliphatischer bzw. aromatischer Amine zum Beispiel der allgemeinen Formel I, II

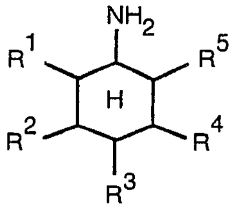

I

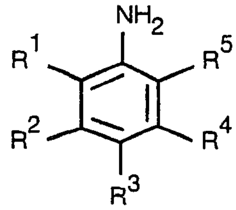

II,

in der $R^1$ bis $R^5$ jeweils ein Wasserstoffatom oder gleiche oder verschiedene oder jeweils zu mehreren gemeinsam einen Substituenten bedeuten können, wird demnach ein entsprechendes Phenol mit Ammoniak und Wasserstoff bei einer Temperatur zwischen etwa 100 und 300, vorzugsweise 100 bis 250°C an einem Träger-Katalysator umgesetzt, der Ruthenium, Rhodium oder Platin und einen basischen Stoff und/oder ein Element aus der Gruppe 1 B, 2 B, 7 B des Periodensystems oder Eisen, Kobalt oder Nickel enthält.

Aliphatische Gruppen mit 1 bis 20 Kohlenstoffatomen, cycloaliphatische Gruppen mit 5 bis 20 Kohlenstoffatomen, Arylgruppen mit 6 bis 20 Kohlenstoffatomen oder Aralkyl- oder Alkylarylgruppen mit 7 bis 20 Kohlenstoffatomen sind Beispiele für derartige Substituenten. Diese können gegenbenenfalls Sauerstoff und/oder Stickstoff als Heteroatome enthalten oder es können zwei Substituenten durch eine Molekülbrücke miteinander verbunden sein. Die Erfindung wird lediglich dadurch beschränkt, daß das als Ausgangsstoff vorgesehene Phenol bei der Reaktionstemperatur einen ausreichenden Dampfdruck besitzen muß, d.h. im günstigsten Falle einen Siedepunkt unterhalb der Reaktionstemperatur.

Mit den erfindungsgemäßen Katalysatoren kann man je nach Reaktionstemperatur und Wasserstoffdruck entweder cycloaliphatische oder aromatische Amine erhalten. Für den Ammoniak gilt, daß er in ausreichender Menge — z.B. 5 bis 10 molaren Überschuß angeboten werden soll. Die Bildung von Nebenprodukten kann fast vollständig unterdrückt werden; empfindliche Substituenten, wie Alkoxygruppen, werden im allgemeinen nicht angegriffen.

Dieser Befund ist überraschend, da nach dem Stande der Technik bei höherer Temperatur zahlreiche Nebenreaktionen und somit Nebenprodukte, wie Cyclohexanol, Cyclohexanon, Dicyclohexylamin, Phenylcyclohexylamin und Diphenylamin zu erwarten sind.

Die ggf. erhaltenen Cyclohexylamine lassen sich bei Bedarf leicht in Aniline überführen. Cyclohexylamine und entsprechende Aniline lassen sich im übrigen leicht trennen, so daß Mischungen ohne weiteres in Kauf genommen werden können. Auf dieser Tatsache beruht ein wesentlicher Vorteil des hier beschriebenen Verfahrens, denn die als Ausgangsstoffe verwendeten Phenole lassen sich vollständig umsetzen.

Die als Ausgangsstoffe verwendeten Phenole sind im allgemeinen gut zugängliche Verbindungen (Houben-Weyl, Methoden, Band 6/Ic),

Entsprechend der o.a. Formel für die Zielprodukte kann ein entsprechendes Phenol Substituenten $R^1$ bis $R^5$ haben, die die vorstehende Bedeutung haben. $R^1$ bis $R^5$ können ferner gleiche oder verschiedene Substituenten und selbstverständlich Wasserstoff bedeuten, wobei Alkyl- oder Aralkylreste mit jeweils 1 bis 20 C-Atomen Kettenlänge besonders wichtige Substituenten darstellen. Die Substituenten können außerdem Sauerstoff- oder Stickstoffatome in der Kette oder in heterocyclischer Anordnung aufweisen.

Zwar ist ist die Umsetzung von Hydroxybenzol ("Phenol") selbst nicht wirtschaftlich interessant, aber sie ist technisch möglich. Andere, substituierte Phenole sind etwa o-, m- und p-Kresol, o-Ethylphenol, o-n-Butylphenol, o-sek.-Butylphenol, 2,4-Dimethylphenol, 2,6-Dimethylphenol, 2,3,6-Trimethylphenol, 2,4,6-Trimethylphenol, 2-Cyclohexylphenol, 2,6-Dimethyl-3-phenyl-phenol, 2,6-Diethylphenol, 2,5-Diisopropylphenol, 2,6-Di-tert.-butylphenol, 2-Methyl-6-sek.-butylphenol, 3-tert.-Butylphenol, α-Naphthol, β-Naphthol, Bisphenol A (=2,2-Di-(p-Hydroxyphenyl)-propan).

Der erforderliche Ammoniak kann — bezogen auf das eingesetzte Phenol — in stöchiometrischer Menge oder in einem, vorzugsweise 2 bis 10-fachen oder auch höheren Überschuß angewendet werden.

Das Verfahren kann kontinuierlich (fortlaufend) oder diskontinuierlich (absatzweise) durchgeführt werden. In jedem Falle kann der Katalysator in einem Festbett oder als Suspension angeordnet sein. Bevorzugt bei kontinuierlicher Arbeitsweise ist ein fest angeordneter Katalysator. Bei kontinuierlicher Arbeitsweise wird der Ammonaik zusammen mit dem Wasserstoff und dem Dampf des umzusetzenden Phenols über den Katalysator geleitet.

Das nach dem Verfahren der Erfindung verwendete Katalysatorsystem enthält Ruthenium, Rhodium oder Platin auf einem Träger, wobei aus wirtschaftlichen Grüden Rhodium und Platin gegenwärtig weniger in Betracht kommen. Für die Herstellung cycloaliphatischer Amine ist die Verwendung von Ruthenium besonders günstig.

Die Metalle können auf dem Träger allein verwendet werden, günstig ist aber die Verwendung gewisser aktiver Zusätze, nämlich Elementen bzw. Verbindungen von Elementen der Gruppen 1B, 2B und 7B des Periodensystems sowie Eisen, Kobalt und Nickel, unabhängig hiervon die Verwendung basischer Stoffe, d.h. Verbindungen der ersten bis dritten Hauptgruppe des Periodensystems. Die letzteren, basischen Stoffe bilden vorzugsweise einen Bestandteil des Trägers. Natürlich können die Zusätze allein oder im Gemisch miteinander neben dem Edelmetall vorliegen. Die aktiven Bestandteile des Katalysators

sind auf einem Träger aufgebracht, der aus Aluminiumoxid oder einem unter Mitwirkung von Aluminiumoxid aufgebauten Stoff besteht. Aluminiumsilikat oder Spinelle des Aluminiums sind solche Stoffe. Die Zusätse, die dem Katalysator seine Eigenschaften verleihen, können sich an der Oberfläche des Katalysators befinden, zusammen mit dem übrigen Aluminiumoxid usw. in Form einer Mischung den Träger darstellen oder mit dem ursprünglichen Träger durch nachträgliches Tempern ein gemeinsames Kristallgitter bilden (beispielsweise bei Aluminiumoxidträgern Bildung von typischen Spinellgittern mit bestimmten Metallen). Das bedeutet, daß der Träger Aktivität und auch Lebensdauer des Katalysators günstig beeinflussen kann.

Als Zusätze kommen in Frage:

a) Basische Zusätze, wie Oxide, Hydroxide oder Carbonate der Alkalimetalle, vorzugsweise des Lithiums und Natriums, der Erdalkalimetalle, vorzugsweise des Magnesiums und Calciums und der Seltenerdmetalle, vorzugsweise des Cers und Praseodyms (Neodyms).

b) Weitere Metalle, wie Eisen, Nickel, Kobalt, Mangan, Zink, Cadmium und Silber.

Die Zusätze können gemeinsam mit dem Edelmetall durch Tränken des Trägermaterials mit Lösungen von z.B. Nitraten, Chloriden, Formiaten oder Oxalaten aufgebracht werden. Durch anschließende theremische Behandlung, die gewöhnlich zwischen 400 und 600°C durchgeführt wird, erfolgt Überführung in die Oxidform. Soll bei Aluminiumoxidträgern mit geeigneten Metallen (Mg, Zn, Co, Mn, Li) Spinellbildung erzielt werden, muß nach der Tränkung auf eine Temperatur von 900—1300°C erhitzt werden (siehe Ullmanns Encyklopädie der technischen Chemie, 3. Auflage (1955) Band 6, S. 242—244, Gmelin, System-Nr. 35, Al, Tl, 1934—1935, S. 26—28) und anschließend in der üblichen Weise das Edelmetall aufgebracht werden.

Manche Zusätze, wie beispielsweise Calciumoxid oder Magnesiumoxid lassen sich mit einem Träger wie Aluminiumoxid mischen und bilden dann nach gemeinsamem Tempern bei 400—600°C einen neuen Träger, auf dem das Edelmetall niedergeschlagen werden kann.

Der Edelmetallgehalt des Katalysators, bezogen auf das Trägermaterial, liegt gewöhnlich zwischen 0,05 bis 15 Gew.%.

Das Gewichtsverhältnis der Zusätze zum Edelmetall kann unterschiedlich sein; es kann z.B. zwischen 10 000:1 bis 1:50, bevorzugt bei 100:1 bis 1:50 liegen. Man verwendet den Katalysator beispielsweise in Form von Strängen mit einem Durchmesser von 3 mm und einer Länge von 10 mm oder als Pulver, je nach dem vorgesehenen Anwendungszweck. Cyclohexylamin und substituierte Cyclohexylamine finden Verwendung auf dem Textilhilfsmittelsektor, Pflanzenschutzmittelsektor, als Korrosionsschutzmittel und als Monomere für Kunststoffe (Ullmanns Encyklopädie der techn. Chemie, 3. Auflage, Band 5, Seiten 681—685).

Herstellmöglichkeiten für den Katalysator:

a) Auf strang- oder pulverförmiges γ-Aluminiumoxid wird das Edelmetall zusammen mit dem Zusatz (Mangan, Zink, Silber, Seltenerdmetalle u.a.) in der gewünschten Menge durch Tränken mit beispielsweise deren Nitratlösungen und anschließendes Eindampfen bis zur Trockne aufgebracht. Danach wird 6 Stunden bei 550°C getempert und im Wasserstoffstrom bei 300°C reduziert.

b) Auf strang- oder pulverförmiges γ-Aluminiumoxid wird zunächst die gewünschte Menge des Zusatzes (Mangan, Zink, Cobalt, Magnesium, Lithium u.a.) durch Tränken mit entsprechenden wäßrigen Nitrat- oder Formiatlösungen aufgebracht und bei 150°C getrocknet. Der derart vorbehandelte Träger wird nun entweder 6 Stunden lang auf 550°C erhitzt oder, wenn Spinellbildung erreicht werden soll, 6 Stunden lang bei 1050°C geglüht. Nun wird der Träger mit wäßriger Edelmetallnitratlösung getränkt und durch 7-stündiges Erhitzen bei 300°C im Wasserstoffstrom reduziert. Wurde zum Tränken eine Lösung eines Chlorids genommen, wird mit alkalischer Formalinlösung reduziert.

c) γ-Aluminiumoxid und ein basisches Oxid (MgO, CaO) werden in dem gewünschten Mengenverhältnis intensiv miteinander vermischt. Diese Mischung wird 6 Stunden lang auf 450°C erhitzt, anschließend mit Edelmetallnitratlösung getränkt und 7 Stunden mit Wasserstoff bei 300°C reduziert. Der Katalysator wird mit einer 5%igen wäßrigen Hydrazinhydratlösung nachreduziert und dann bei 120°C getrocknet.

Die in den nachfolgenden Beispielen angegebenen Siedepunkte beziehen sich, sofern nicht anders angegeben, auf atmosphärischen Druck, sonst auf verminderten Druck in mbar.

Beispiel 1

In einen Rohrreaktor mit einem Fassungsvermögen von 1 l wird ein Katalysator (Stränge mit 4 mm Durchmesser, 10 mm Länge), der 0,5 Gew.-% Rhodium auf Mangnesium-Aluminiumspinell enthält, eingefüllt und auf 120°C erhitzt. Hierüber werden stündlich bei Atmosphärendruck 50 g 2,6-Dimethylphenol geführt. Gleichzeitig wird eine Gasmischung aus 200 Nl Wasserstoff und 100 Nl Ammoniak pro Stunde im Gleichstrom hindurchgeleitet. Das Reaktionsprodukt wird — sobald es den Reakto verläßt — abgekühlt. Es besteht nach gaschromatographischer Analyse aus 98,6 Gew.-% 2,6-Dimethylcyclohexylamin. Nach einer destillativen Aufarbeitung des Reaktionsproduktes erhält man aus f100 g Ausgangsstoff 103 g 2,6-Dimethylcyclohexylamin (Kp=167 bis 168°C), entsprechend einer Ausbeute von 97% d.Th.

Beispiel 2

Man verfährt analog Beispiel 1, verwendet aber einen Katalysator, der 1,5 Gew.-% Ruthenium auf Zink-Aluminiumspinell enthält und eine Reaktionstemperatur von 180°C. Ausgehend von m-(4-Methyl-tetrahydropyran-2-yl)-phenol erhält man als Reaktionsprodukt 3-(4'-Methyl-tetrahydropyran-2'-yl)-cyclohexylamin (Kp=80 bis 81°C/0,13 mbar) in einer Ausbeute von 94% d.Th.

Beispiel 3

Man verfährt analog Beispiel 1, verwendet aber einen Katalysator, der 0,5 Gew.-% Ruthenium auf Lithium-Aluminiumspinell enthält und eine Reaktionstemperatur von 160°C. Ausgehend von 2-Methyl-6-tert.-butylphenol erhält man als Reaktionsprodukt 2-Methyl-6-tert.-butyl-cyclohexylamin (Kp=220 bis 221°C) in einer Ausbeute von 94% d.Th.

Beispiel 4

Man verfährt analog Beispiel 1, verwendet aber einen Katalysator, der 1,5 Gew.-% Rhodium auf Zink-Aluminiumspinell enthält und eine Reaktionstemperatur von 180°C. Ausgehend von 2,2-Dimethyl-5-hydroxychroman erhält man als Reaktionsprodukt 2,2-Dimethyl-5-amino-hexahydrochroman (K-=128 bis 131°C/27 mbar) in einer Ausbeute von 94% d.Th.

**Patentansprüche**

1. Verfahren zur Herstellung von cycloaliphatischen und/oder aromatischen Aminen der allgemeinen Formel $RNH^2$, in der R einen gegebenenfalls substituierten oder annellierten Cycloalkyl- oder Phenylrest bedeutet, durch Umsetzung von entsprechenden aromatischen Hydroxyverbindungen (Phenolen) mit Ammoniak und Wasserstoff in Gegenwart eines Katalysators bei erhöhter Temperatur, dadurch gekennzeichnet, daß man die Umsetzung in Gegenwart eines auf einem Träger aufgebrachten Ruthenium-, Rhodium- oder Platinkatalysators, der einen basischen Stoff und/oder ein Element aus der Gruppe 1 B, 2 B, 7 B des Periodsystems, Eisen, Kobalt oder Nickel enthält, in der Gasphase durchführt.

2. Verfahren nach Anspruch 1, dadurch gekennzeichnet, daß man die Umsetzung bei einem Druck von unter 10 bar ausführt.

3. Verfahren gemäß Anspruch 1, dadurch gekennzeichnet, daß der Katalysator als basischen Stoff ein Oxid, Hydroxid oder Carbonat eines Alkalimetalls oder eines Erdalkalimetalls enthält.

4. Verfahren gemäß Anspruch 1, dadurch gekennzeichnet, daß der Katalysator als basischen Stoff ein Oxid, Hydroxid oder Carbonat eines Seltenerdmetalls enthält.

5. Verfahren nach Anspruch 1, dadurch gekennzeichnet, daß daß der Katalysator eine Magnesiumverbindung enthält.

6. Verfahren nach Anspruch 4, dadurch gekennzeichnet, daß der Katalysator eine Praseodym/Neodym- oder Cerverbindung enthält.

7. Verfahren nach einem der vorstehenden Ansprüche 1 bis 6, dadurch gekennzeichnet, daß man zur Gewinnung eines aromatischen Amins das als Zwischenprodukt angefallene oder unabhängig erhaltene Cyclohexylamin bei 180 bis 300°C umsetzt.

**Revendications**

1. Procédé pour la préparation d'amines cycloaliphatiques et/ou aromatiques de formule générale $RNH^2$, dans laquelle R représente un radical cycloalkyle ou phényle éventuellement substitué ou à noyaux condensés, par réaction de composés hydroxy aromatiques (phénols) correspondants avec l'ammoniac et l'hydrogène en présence d'un catalyseur à température élevée, caractérisé en ce qu'on mène la réaction en phase gazeuse, en présence d'un catalyseur de ruthénium, rhodium ou platine fixé sur un support, contenant une substance basique et/ou un élément des groupes 1B, 2B, 7B du système périodique, du fer, du cobalt ou du nickel.

2. Procédé selon la revendication 1, caractérisé en ce qu'on mène la réaction sous une pression de moins de 10 bars.

3. Procédé selon la revendication 1, caractérisé en ce que le catalyseur contient, en tant que substance basique, un oxyde, hydroxyde ou carbonate d'un métal alcalin ou d'un métal alcalinoterreux.

4. Procédé selon la revendication 1, caractérisé en ce que le catalyseur contient, en tant que substance basique, un oxyde, hydroxyde ou carbonate d'un métal des terres rares.

5. Procédé selon la revendication 1, caractérisé en ce que le catalyseur contient un composé du magnésium.

6. Procédé selon la revendication 4, caractérisé en ce que le catalyseur contient un composé du praséodyme/néodyme ou du cérium.

7. Procédé selon l'une quelconque des revendications 1 à 6, caractérisé en ce que pour l'obtention d'une amine aromatique, on fait réagir à une température de 180 à 300°C la cyclohexylamine formée en tant que produit intermédiaire ou obtenue indépendamment.

**Claims**

1. A process for the production of cycloaliphatic and/or aromatic amines of the general formula $RNH^2$, where R denotes a cycloalkyl or phenyl radical which may be substituted or fused, by reacting corresponding aromatic hydroxy compounds (phenols) with ammonia and hydrogen in the presence of a catalyst at elevated temperature, characterized in that the reaction is carried out in the gas phase in the presence of a supported ruthenium, rhodium or platinum catalyst containing a basic substance and/or an element of Group 1B, 2B, or 7B of the Periodic System, iron, cobalt or nickel.

2. A process as claimed in claim 1, characterized in that the reaction is carried out at a pressure of less than 10 bar.

3. A process as claimed in claim 1, characterized in that the catalyst contains an oxide, hydroxide or carbonate of an alkali metal or alkaline earth metal as the basic substance.

4. A process as claimed in claim 1, characterized in that the catalyst contains an oxide, hydroxide or carbonate of a rare-earth metal.

5. A process as claimed in claim 1, characterized in that the catalyst contains a magnesium compound.

6. A process as claimed in claim 4, characterized in that the catalyst contains a praseodymium/neodymium compound or a cerium compound.

7. A process as claimed in any of the preceding claims 1 to 6, characterized in that for the preparation of an aromatic amine the cyclohexylamine obtained as an intermediate or independently is reacted at 180°C to 300°C.